Europäisches Patentamt

(19) European Patent Office

Office européen des brevets

(11) Publication number: **0 170 169 B1**

(12) **EUROPEAN PATENT SPECIFICATION**

(45) Date of publication of patent specification: **20.03.91**    (51) Int. Cl.⁵: **C12N 15/38**, C12P 21/02, A61K 39/245, C12N 1/18, //C12R1/865

(21) Application number: **85109042.3**

(22) Date of filing: **19.07.85**

(54) Recombinant DNA containing a herpes simplex virus gene or a fragment thereof, yeast transformed with said recombinant DNA, and method for the production of herpes simplex virus proteins.

(30) Priority: **20.07.84 JP 151766/84**
**11.12.84 JP 262465/84**

(43) Date of publication of application:
**05.02.86 Bulletin 86/06**

(45) Publication of the grant of the patent:
**20.03.91 Bulletin 91/12**

(84) Designated Contracting States:
**AT BE CH DE FR GB IT LI NL SE**

(56) References cited:
**EP-A- 0 100 521**
**EP-A- 0 101 655**
**EP-A- 0 105 149**
**EP-A- 0 133 063**

(73) Proprietor: **Juridical Foundation The Chemo-Sero-Therapeutic Research Institute**
**668, Okubo Shimizu-machi**
**Kumamoto-shi Kumamoto-ken(JP)**

(72) Inventor: **Nozaki, Chikateru**
**622-8, Shinchi Shimizu-machi**
**Kumamoto-shi Kumamoto-ken(JP)**
Inventor: **Makizumi, Keiichi**
**137, Yamamuro Shimizu-machi**
**Kumamoto-shi Kumamoto-ken(JP)**
Inventor: **Kino, Yoichiro**
**2-142, Musashigaoka**
**Kumamoto-shi Kumamoto-ken(JP)**
Inventor: **Eto, Tatsuo**
**29-27, Tsunoura-machi**
**Kumamoto-shi Kumamoto-ken(JP)**
Inventor: **Ohtomo, Nobuya**
**6-22-29, Shimasaki**
**Kumamoto-shi Kumamoto-ken(JP)**

(74) Representative: **Vossius & Partner**
**Siebertstrasse 4 P.O. Box 86 07 67**
**W-8000 München 86(DE)**

## Description

The present invention relates to a novel recombinant DNA containing a herpes simplex virus gB gene or a fragment thereof, a yeast transformed with said recombinant DNA, and a method for the production of herpes simplex virus gB proteins, wherein a genetic engineering technique is applied to the production of the desired herpes simplex virus proteins which have a high purity and are useful for the production of herpes simplex vaccine effective for the prophylaxis of herpes simplex virus infections.

More particularly, the present invention provides a novel recombinant DNA which is obtained by inserting a or a fragment thereof HSVgB gene, or a fragment thereof into a shuttle vector which can replicate in both Escherichia coli and yeast downstream of the expression control region of the repressible acid phosphatase gene (said region being hereinafter referred to as "acid phosphatase promoter" or "acid phosphatase gene") carried by the vector, a novel transformed yeast which is produced by transforming a yeast with said recombinant DNA, and a method for the production of gB HSV proteins in a high yield and with high purity by culturing said transformed yeast in a suitable medium.

In developed countries, population having immunity against HSV has recently decreased, and hence, the frequency of serious HSV infections such as herpes genitalis, neonatal herpes infection and herpes encephalitis increased in these countries. In order to prevent such HSV infections, vaccines are useful. There have already been proposed some vaccines such as attenuated vaccines comprising attenuated HSV and inactivated vaccines containing HSV DNA. It is known, however, that HSV includes some problems such as latent infection and carcinogenicity, and that the conventional attenuated vaccines and inactivated vaccines include such side effects and hence are not suitable in practical viewpoint.

Cells infected by HSV produce several glycoproteins [e.g. gB, gC, gD, gE, etc., the nomenclature of gA and gB has been standardized as "gB" in International Herpes Virus Workshop (Oxford, England) in 1983]. Since it has been found that these glycoproteins show important functions as an antigen for inhibiting HSV infection, various studies have been done on component vaccines comprising these glycoproteins. For instance, Cappel et al. have reported that glycoproteins extracted from HSV-infected cells or virus particles are effective as an antigen for preventing HSV infections [cf. Cappel et al., Arch. Virol., 73 , 61 (1982)]. However, the component vaccine comprising such glycoproteins extracted from HSV-infected cells or virus particles contains plenty of proteins origined from the host cells and bars the problem of side effects due to the extra proteins. In order to obtain suitable component vaccine having no side effects, it is necessary to obtain highly purified glycoproteins. The present inventors aimed at gB which is one of the glycoproteins and have experimentally confirmed by an experiment in mice that the highly purified gB is very effective [cf. Kino, Cellular Technology, 3 , 120 (1984)].

The glycoprotein gB- is usually produced by inoculating a virus into culture cells and then culturing the cells. However, this method is troublesome in the procedure because of handling infectious material and in the complicated steps, and further, it is impossible to confirm the complete removal of viral DNA carrying carcinogenic gene. Thus, it is very difficult to produce a practically safe component vaccine from the natural glycoprotein gB.

The present inventors have intensively studied on a safe vaccine containing no carcinogenic gene by isolating the gB gene and expressing said gene or a fragment thereof in yeast. They have accomplished the isolation of the gB gene and then found that when the isolated gB gene or a fragment thereof is recombined into a specific plasmid vector containing yeast gene and E. coli gene and carrying the expression control region of the repressible acid phosphatase gene of yeast under control of said phosphatase promoter, there is obtained the desired recombinant DNA which is useful for transforming a yeast. When this transformed yeast is cultured under suitable conditions it can produce the desired HSV gB proteins. The present inventors have further found that when a part of the 3'-region (tail) of the gB gene is removed by treating the gB gene with the restriction enzyme Sac I and the resulting DNA fragment containing about 90 % of the total gB gene is inserted into the Sac I site of the above plasmid vector, there is obtained a new recombinant DNA which is useful for transforming a yeast. The obtained transformed yeast can express the HSVgB proteins as a chimeric protein which shows gB activity and is useful for the preparation of herpes simplex virus vaccines.

An object of the present invention is to provide a recombinant DNA containing a HSV gB gene according to claim 1 which is suitable for transforming a yeast. Another object of the invention is to provide a yeast transformed with said recombinant DNA. A further object of the invention is to provide a method for the production of HSV gB proteins, preferably chimeric Proteins, using said transformed yeast. These objects and advantages of the present invention will be apparent to persons skilled in the art from the following description.

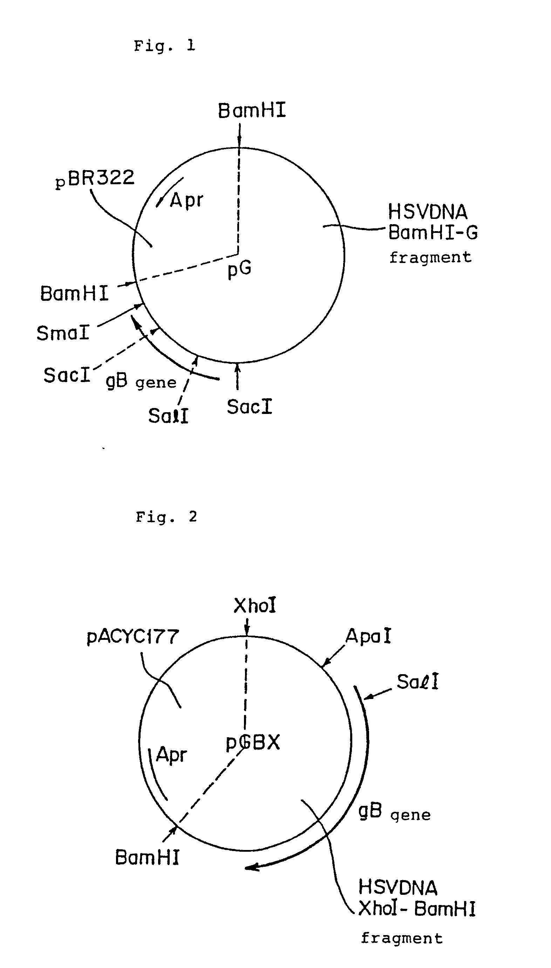

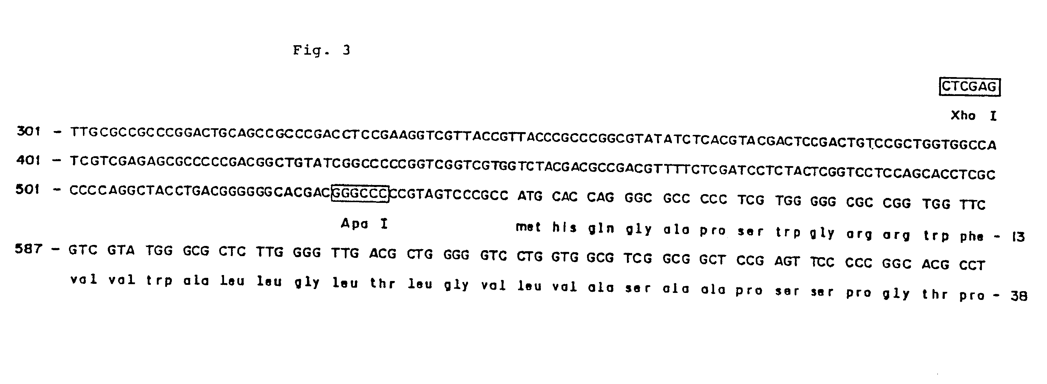

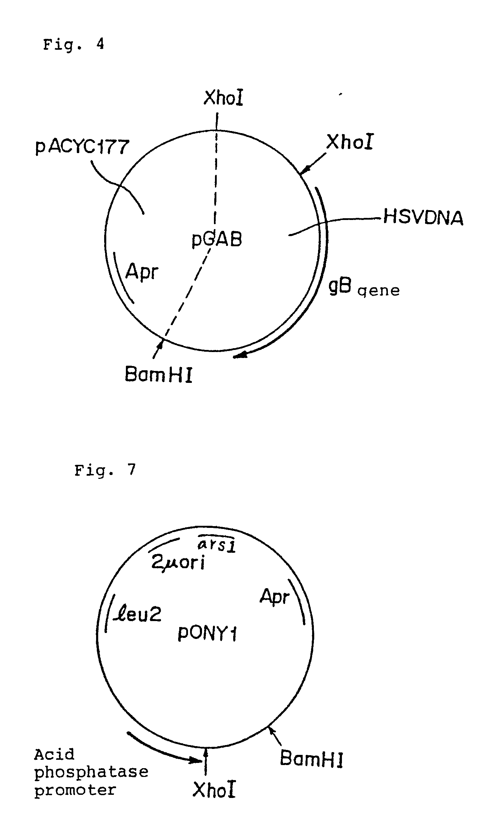

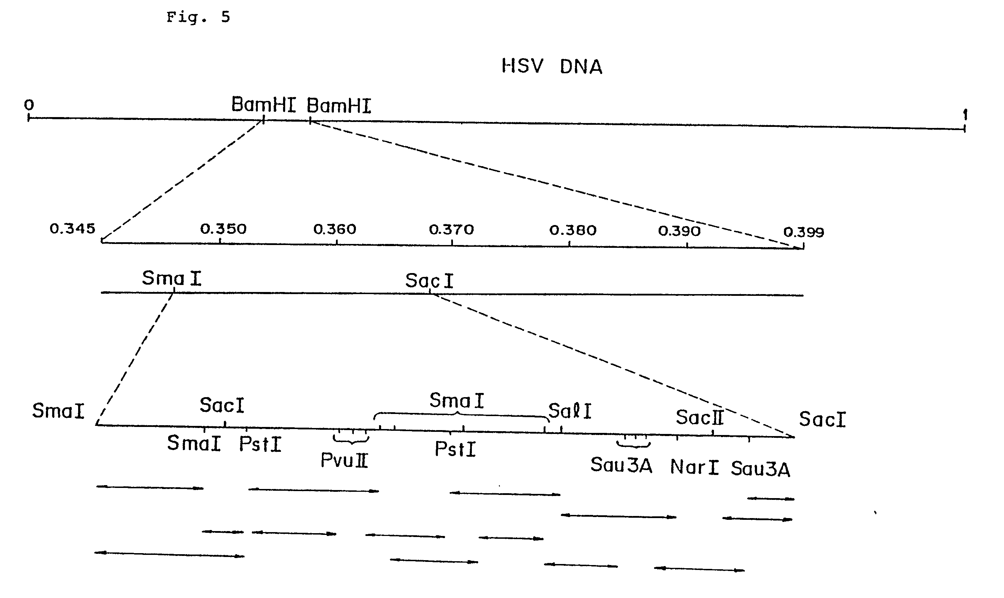

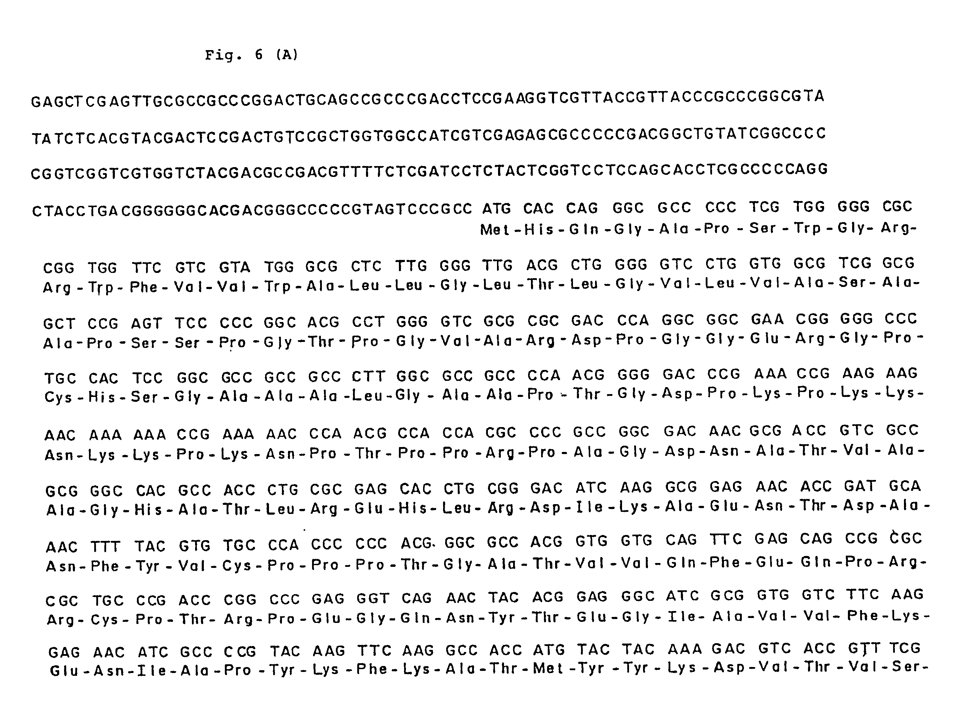

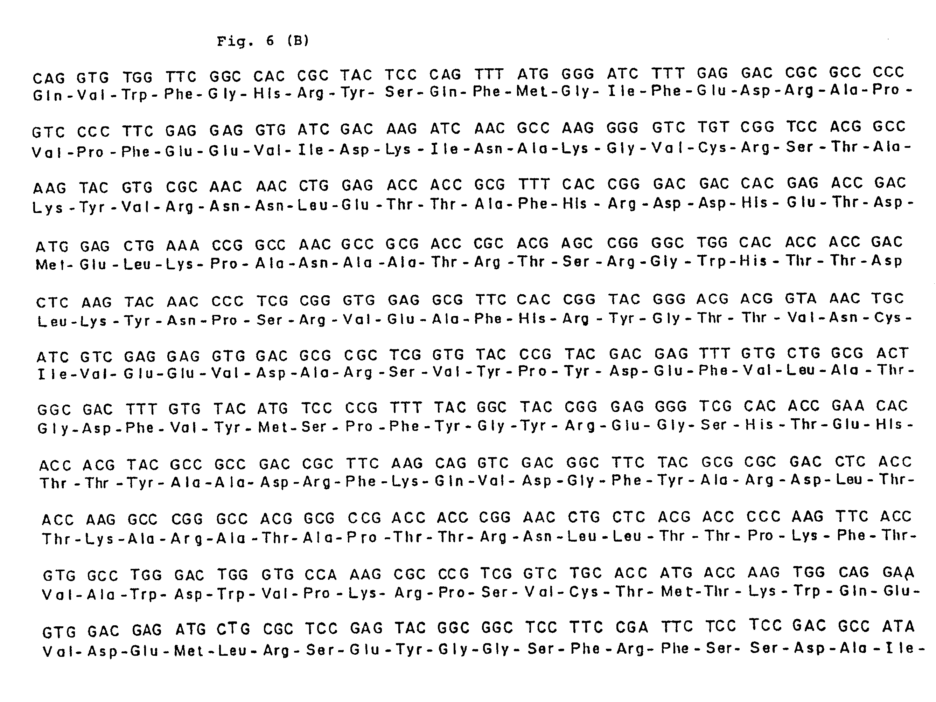

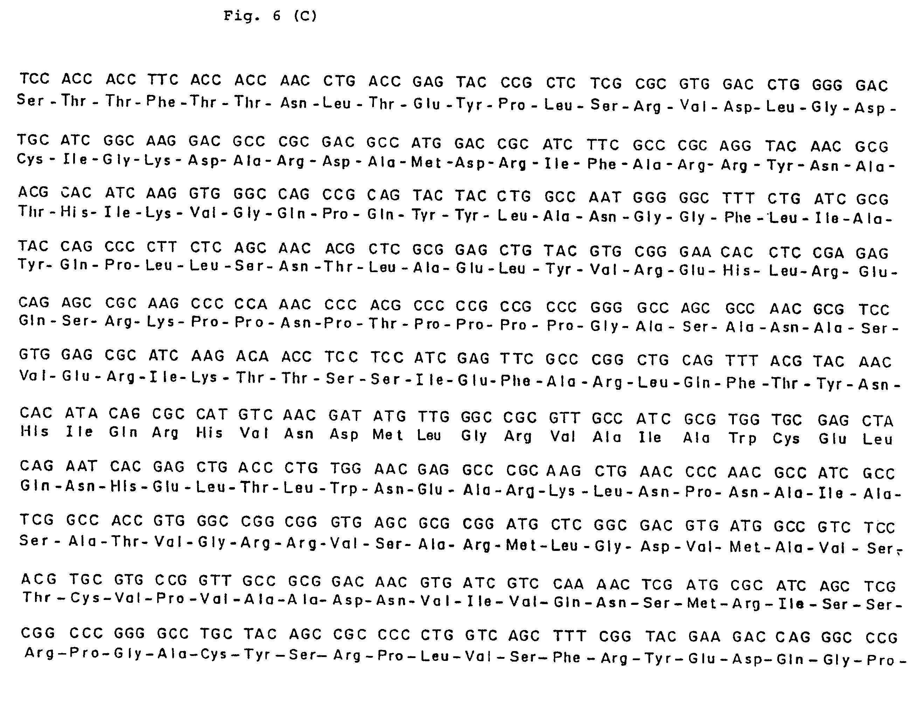

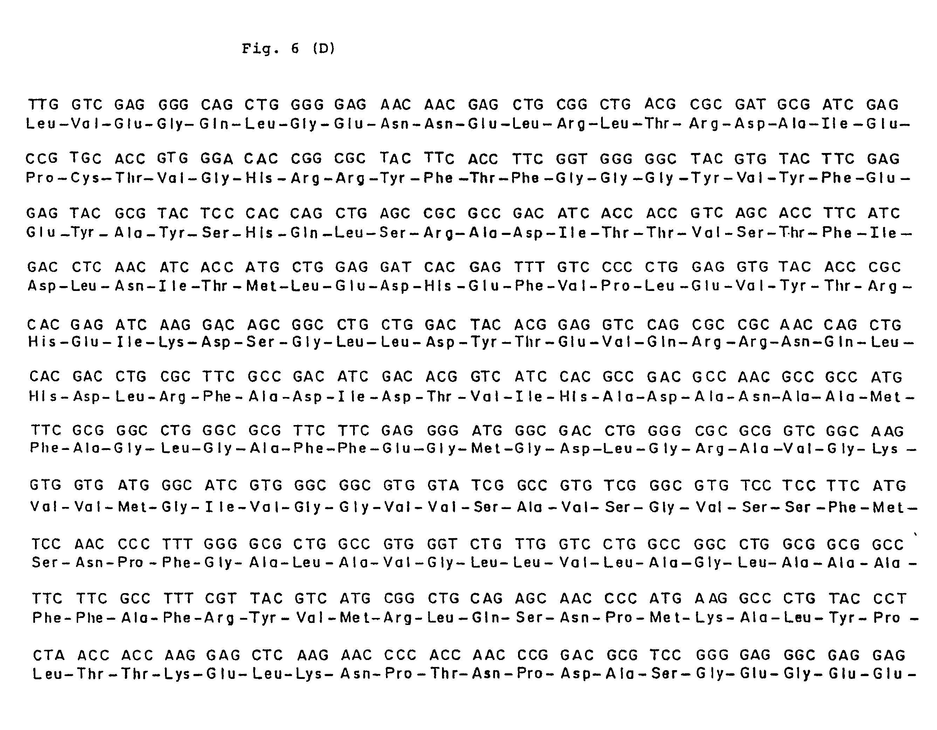

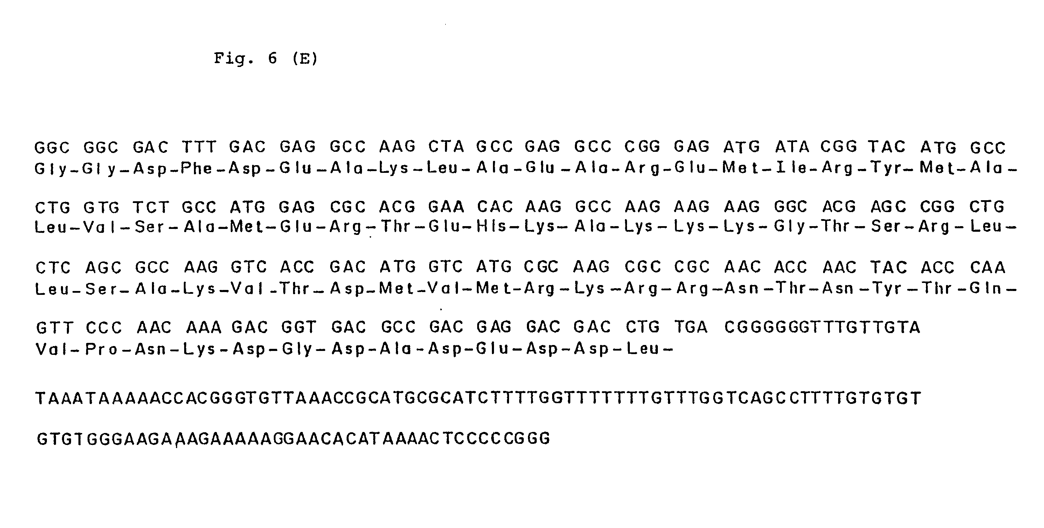

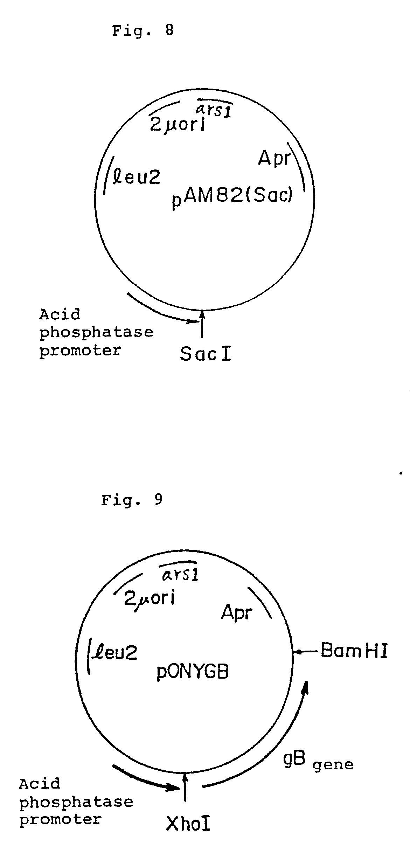

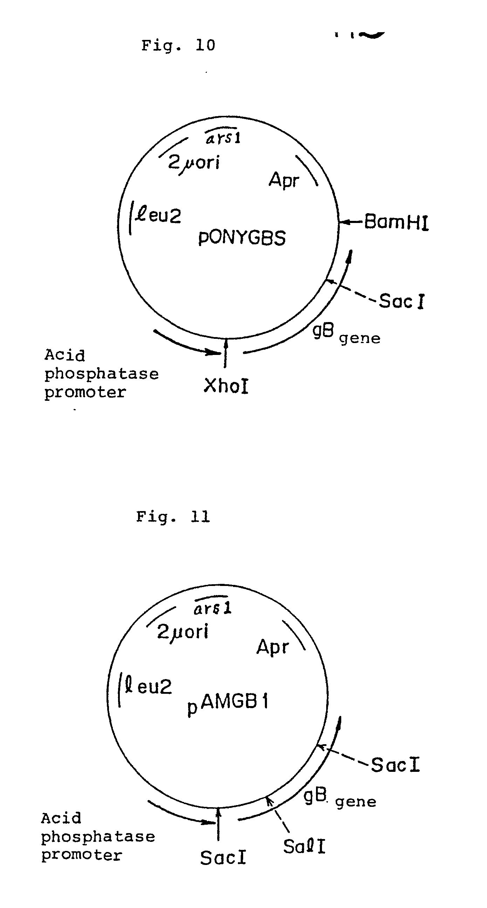

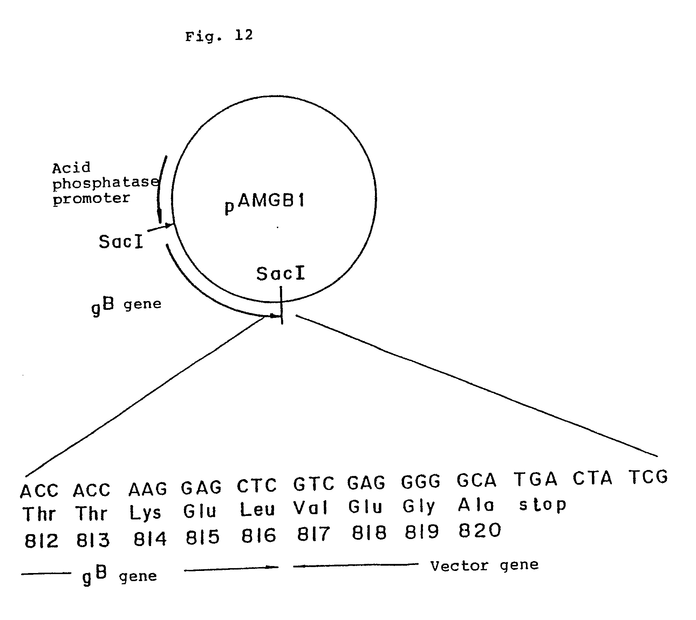

Fig. 1 shows the structure of plasmid pG containing the BamHI-G fragment of HSV DNA. Fig. 2 shows the structure of plasmid pGBX. Fig. 3 shows the base sequence of the HSVgB gene and the upstream region thereof. Fig. 4 shows the structure of plasmid pGAB which is obtained by replacing the Apa I site of plasmid pGBX with an Xho I site. Fig. 5 shows a restriction enzyme map of the region containing the gB gene. Fig. 6 (A) - (E) show the base sequence of/HSVgB gene. Fig. 7 shows the structure of shuttle vector pONYJ. Fig. 8 shows the structure of plasmid pAM 82 (sac). Fig. 9 shows the structure of recombinant plasmid pONYGB containing theHSVgB gene of the present invention. Fig. 10 shows the structure of recombinant plasmid pONYGBS wherein a part of the 3'-region of the gB gene is removed. Fig. 11 shows the structure of plasmid pAMGB1 wherein about 10% of the 3'-region of the gB gene is removed. Fig. 12 shows the base sequence of the gB gene - vector recombination site of the plasmid -pAMGB1.

The desired recombinant DNA of the present invention can be produced using a specific plasmid vector containing a yeast gene and an E. coli gene and carrying the expression control region of the repressible acid phos phatase gene of yeast and then recombining the HSVgB gene, losing about 10 % of the 3'-region of the gene into said vector under control of the phosphatase promoter. The recombinant DNA thus produced is used for transformation of a yeast. When the transformed yeast is cultured under suitable culture conditions, preferably under the conditions that the acid phosphatase promoter is not repressed, the desired HSV gB proteins and HSV gB chimeric proteins are synthesized.

The recombinant DNA, transformed yeast and the production of HSV gB proteins of the present invention are illustrated in more detail below with reference to preferred embodiments thereof.

(1) Production of HSVgB gene-containing fragments

The HSVgB gene used in the present invention is the HSVgB DNA which is cloned in E. coli .

(A) As to the gB gene of HSV (KOS strain: P.S.E.B.M., 1964, Vol. 115) the position on the virus DNA (0.348 - 0.366 map units) and the base sequence have been determined by Bjik et al. [cf. David J. Bjik et al., Virology, 133 , 301 - 314 (1984)].

The HSVgB gene to be inserted into a shuttle vector in the present invention is present in a fragment having about 8 kb (0.345 - 0.399 map units) which is obtained by treating HSV DNA with the restriction enzyme BamHI, said fragment being hereinafter referred to as "BamHI-G fragment".

The HSVgB-containing fragment can be prepared by cleaving HSV DNA with BamHI, and cloning the obtained BamHI-G fragment as follows.

Virus DNA is isolated from HSV which has been grown on Vero cells, the virus DNA is cleaved with the restriction enzyme BamHI, and then the resulting BamHI-G fragment is isolated and extracted by subjecting the BamHI-cleaved HSV-DNA to agarose gel electrophoresis. The BamHI-G fragment is ligated using T₄ ligase into the E. coli plasmid pBR322 which was previously treated with BamHI. E. coli χ1776 is transformed with the above reaction mixture. Among the transformants thus obtained, a strain having ampicillin resistance (Apʳ) and tetracycline sensitivity is selected. The obtained strain is amplified and plasmid PG containing the BamHI-G fragment is isolated. This plasmid pG has the structure shown in Fig. 1.

The plasmid pG thus obtained is cleaved with BamHI and Xho I to give a fragment (3.5 kb) containing the HSVgB gene. The fragment thus obtained is isolated by agarose gel extraction and then is ligated with T₄ ligase into a fragment (2.6 kb) of the E. coli plasmid pACYC177 (Chang, A.C.Y., Cohen, S.N.; J.Bacteriol.134, 1141-1156 (1978)*which was previously treated with BamHI and Xho I. E. coli is transformed with the above reaction mixture, and the resulting transformant having ampicillin resistance is amplified, and then plasmid pGBX containing the HSVgB gene is obtained from * see EP-A-105149 page 17, lines 14-15 the amplified cells. This plasmid pGBX has the structure shown in Fig. 2. According to the dideoxy method [cf. "Proteins, Nucleic acids and Enzymes", Vol. 29, No. 4, 294-306 (1984)], the base sequence of the plasmid pGBX (from the Xho I site to the side of HSV DNA, about 300 bp) is determined (Fig. 3).

This sequence corresponds to the base sequence reported by Bjik et al.

In the above plasmid pGBX, there is about 250 bp from the Xho I site to the translation initiation codon ATG of the gB gene, and at the upstream of ATG, -14 bp to -19 bp, there is an Apa I site. Accordingly, the plasmid pGBX is partially cleaved with the restriction enzyme Apa I and is converted into flush end by treating with DNA polymerase and then re-cyclized with an Xho I linker to give plasmid pGAB wherein only the Apa I site at -14 bp to -19 bp is converted into/Xho I site. This plasmid pGAB has the structure as shown in Fig. 4. The HSV DNA is amplified by cloning in E. coli , and then, it is usually treated with an appropriate restriction enzyme to give a desired fragment. The fragment thus obtained is used for the subsequent construction of the plasmid as mentioned hereinafter.

(B) As to the plasmid pG obtained in the above (A), the base sequence of the Sma I - Sac I region containing gB gene is determined by dideoxy method [cf. "Proteins, Nucleic acids and Enzymes", Vol. 29, No. 4, 294-306 (1984)]. That is, as is shown in the accompanying Fig. 5, the Sma I - Sac I segment is divided into smaller segments by various restriction enzymes, and segements thus obtained (shown by the arrow symbols ← → ) are each inserted into an M 13 vector for determining the base sequence (manufactured by Pharmacia Japan K.K.) and then the resultants are subjected to sequencing.

As a result, as is shown in Fig. 6 (A) - (E), there is an open reading frame of 2712 bp (from ATG to TGA) in the Sma I - Sac I region, and 903 amino acids are encoded. When this base sequence is compared with the base sequence reported by Bjik et al., 6 bases are different (as amino acids, two amino acids are different). Based on the base sequence found above, it is understood that basic amino acids are located at the 3'-region of the gB gene. Accordingly, in order to express the gB gene in yeast, it is preferable to design a vector in which a region other than the 3'-region is inserted downstream of the promoter for expression (i.e. acid phosphatase promoter).

Thus , the above plasmid pG is treated with the restriction enzyme Sac I to remove the 3'-region of the gene to obtain a fragment (2.7 kb), which is hereinafter referred to as "Sac I fragment" . This fragment contains about 90 % of the gB gene and encodes 816 amino acids. It is used for the construction of a plasmid for expressing HSVgB gene as described hereinafter.

(2) Shuttle vector

The shuttle vector used in the present invention is a plasmid vector which contains both a yeast, gene and an E. coli gene and carries the repressible acid phosphatase gene of yeast, e.g. Saccharomyces cerevisiae.

The terms"yeast gene" and "E. coli gene" in this context in principle refer to DNA sequences which are derived from yeast and from E. coli and its plasmids, respectively.

The yeast gene contains a DNA sequence which is necessary for replication of a plasmid in yeast independently from chromosome and contains optionally a gene useful as a selective marker of the transformed yeast. It is found by the present inventors that, as such a DNA sequence for the replication, the combination of ars 1 and 2 μori is quite preferable since the plasmid is provided with an outstanding stability so that the plasmid copy number is very high with the least loss of the plasmid during the transformation and the culturing of the transformed yeast, thereby assuring high production of the desired herpes simplex virus proteins. The selective marker includes, for example, a leucine-producing gene, a histidine-producing gene, a tryptophane-producing gene, a uracil-producing gene, an adenine-producing gene, or the like, which may be used alone or in combination of two or more thereof.

The E. coli gene contains a DNA sequence necessary for the replication of the plasmid within cells of E. coli , for example, a DNA sequence of a replication initiating region of plasmid Col EI, and preferably contains a gene useful as a selective marker of the transformed E. coli . The selective marker includes, for example, an ampicillin-resistant gene, a kanamycin-resistant gene, tetracycline-resistant gene, chloramphenicol-resistant gene, or the like, which may be used alone or in combination of two or more thereof. Commonly used E. coli DNA is pBR322 which contains an ampicillin resistant gene and tetracycline-resistant gene.

The shuttle vector used in the present invention is characteristic in that it carries the repressible acid phosphatase promoter of the yeast. This acid phosphatase promoter is usually a promoter of a gene encoding a polypeptide of 60,000 dalton (P60) which constitutes the phosphatase.

Suitable examples of the shuttle vector are produced from shuttle vector pAT 77 wherein yeast DNA containing ars 1 , 2 μori and a leucine-producing gene (Leu 2) as the yeast gene is combined with E. coli plasmid pBR322, i.e. by treating the shuttle vector pAT 77 with exonuclease BAL 31 to delete a part or whole of the structural gene of acid phosphatase and further optionally various regions upstream therefrom up to -100 bp, preferably from +1 to -50 bp. A representative example is the shuttle vector pAM 82 wherein upstream till -33 bp is deleted. The method for the production of these shuttle vectors is disclosed in Japanese Patent First Publication No. 31799/1984 and EP-A-105149. The shuttle vectors pAT 77 and pAM 82 carried by Saccharomyces cerevisiae (i.e. Saccharomyces cerevisiae AH 22/pAT 77 and Saccharomyces cerevisiae AH 22/pAM 82, respectively) have been deposited at Fermentation Research Institute, Agency of Industrial Science and Technology, Japan under Budapest Treaty as "FERM BP-324" and "FERM BP-313", respectively.

The shuttle vector pAM 82 has an Xho I site downstream of the acid phosphatase promoter and also a Pvu II site further downstream therefrom. In order to insert the HSVgB gene (i.e. Xho I - BamHI fragment) into the vector, pAM 82 is cleaved with the restriction enzyme Pvu II, and a BamHI linker is bound to the resulting flush ends, by which the combined fragment is re-cyclized to form a plasmid pONY1 having a structure as shown in Fig. 7. This plasmid pONY₁ can express a foreign gene in the pure form under the control of acid phosphatase promoter, and the site to be recombined can readily be cleaved by treating it with restriction enzymes BamHI and Xho I, and hence, this shuttle vector is, suitable for recombining genes required.

Besides, when the above shuttle vector pAM82 is cleaved with Xho I in order to recombine it with the HSVgB gene (Sac I fragment, 2.7 kb) and then is converted into flush end with DNA polymerase, and a Sac I linker is bound to said site, by which the combined fragment is re-cyclized , plasmid pAM 82 (Sac) results which has the structure shown in Fig. 8. This plasmid pAM 82 (Sac) can express a foreign gene in the pure form under the control of acid phosphatase promoter, and the site to be recombined can readily be cleaved by treating it with the restriction enzyme Sac I, and inserting a DNA fragment having Sac I-ends.

(3) Construction of recombinant DNA (HSV gene-expression plasmid)

(A) The recombinant plasmid of the present invention, i.e. a plasmid recombined with HSV gene, can be prepared by firstly cleaving the above shuttle vector pONY1 with restriction enzymes BamHI and Xho I, linking thereto the above HSVgB gene [i.e. a fragment (3.3 kb) containing the gB gene which is obtained by cleaving the plasmid pGAB with BamHI and Xho I], and amplifying the resulting plasmid in E. coli to obtain the desired recombinant plasmid pONYGB having the structure shown in Fig. 9.

(B) In order to express a material having gB antigenicity and immunogenicity more effectively, a plasmid in which the gB gene without its 3'-region is inserted is prepared as follows:

The plasmid pONYGB obtained above is cleaved with the restriction enzyme Sac I and is converted into flush ends with DNA polymerase. Thereto the universal translation terminator is ligated : 5'-d-[GCTTAATTAATTAAGC]-3' (sold by Amersham Japan K.K.) and then the plasmid DNA is re-cyclized. The thus obtained plasmid pONYGBS has the structure shown in

Fig. 10, wherein at the Sac I site in the gB gene, the termination codon (TAA) is inserted, and hence, the translation of the m-DNA is stopped at this site and thus about 90 % of gB gene (consisting of 816 amino acids) is expressed. The plasmid wherein about 10 % of the tail region of the gB gene is removed can express a larger amount of proteins having gB antigencity and immunogenicity in comparison with the plasmid pONYGB.

(C) The recombinant plasmid containing the HSV gene without the 3'- region can also be prepared by cleaving the above shuttle vector pAM 82 (Sac) with the restriction enzyme Sac I, linking thereto the above HSVgB gene (Sac I fragment, 2.7 kb), and amplifying the resultant in E. coli to give the desired recombinant plasmid pAMGB1 having the structure shown in Fig. 11.

In this plasmid, the gB gene is inserted downstream of the acid phosphatase promoter, and 816 amino acids are encoded in said gB gene, and thereafter further 4 amino acids are encoded utilizing the base sequence at the vector side in the same frame, and then the frame is stopped at TGA. Thus, totally 820 amino acids are encoded (cf. Fig. 12).

(4) Transformation of yeast

The yeast to be transformed includes a mutant strain of yeast which is complemental with the selective marker gene of the transformed yeast carried on the plasmid, for example, a leucine-requiring mutant, Saccharomyces cerevisiae AH 22 [a, leu 2, his 4, Can 1 (Cir⁺)]' (cf. Hinnen, A. et al., Proc. Natl. Acad. Sci., U.S.A., 75 , 2157-2161 , 1978) or Saccharomyces cerevisiae AH 22 pho 80 [a, leu 2, his 4, Can 1 (Cir⁺)] (cf. Thoe, A. et al., J. Bacteriol., 145 , 221-232, 1981). After amplifying by E. coli, the recombinant plasmid is applied to the mutant strain of yeast in a usual manner, for example, by mixing the plasmid DNA with cells obtained by converting into spheroplast, followed by mixing cells treated with calcium and the plasmid DNA, by which the transformation is effected. The desired transformed yeast is selected and isolated from the yeast culture thus treated based on the expression of a gene complemental with the mutation of the host yeast carries on the vector, for example, expression of a leucine-producing gene.

In addition to the above-mentioned leucine-requiring strain, various other mutant strains such as a histidine-requiring strain, tryptophane-requiring strain, uracil-requiring strain, adenine-requiring strain, or the like can be used as the yeast.

EP 0 170 169 B1

(5) Culture of transformed yeast and production of HSVgB

The transformed yeast obtained above is cultured in a medium containing phosphoric acid in a usual manner, the culture cells in logarithmic growth phase are transferred to an inorganic phosphate free medium and then are cultured under a condition that the acid phosphatase promoter is not repressed. After the culture, the produced cells are collected and lysed in a usual manner to give a lysed cell solution containing a large amount of the desired HSVgB [HSV chimeric proteins in case of using the yeast transformed with the plasmid pAM 82 (Sac)].

Depending on the kind of an yeast, for instance, when pho 80 mutant strain is used, the culture is not necessarily required to be carried out under the condition that the acid phosphatase promoter is not repressed, but may be done under a usual condition to give directly the desired HSVgB or HSV chimeric proteins in a large amount.

The HSVgB or HSV chimeric proteins can be purified by conventional purification methods. For instance, the proteins-containing solution is passed through a column packed with a gel bound with an anti-gB antibody, and then the adsorbed gB is eluted with 3 M KSCN.

The gB thus obtained has the same immunological properties as those of the natural gB obtained from HSV-infected cells and can be used for the preparation of HSV vaccine and diagnostic reagents.

The recombinant DNA, transformed yeast, and the production of HSVgB of the present invention are illustrated by the following example, but should not be construed to be limited thereto.

Example

(1 ) Preparation of DNA containing HSVgB gene:

(i) Preparation of HSV-1 DNA:

Vero cells (about $5 \times 10^8$ cells) is infected with 0.5-1 PFU/cell of HSV type 1 (HSV-1 ) KOS strain and the cells are culured at 37°C for 20-24 hours. When the cells are sufficiently modified, the culture mixture is centrifuged at 28,000 r.p.m. for 1 hour to separate the infected cells and the supernatant, and thereby pellets of infected cells (2-3 ml) are isolated. The pellets are suspended in a phosphate buffered saline solution (hereinafter, referred to as "PBS") (pH 7.2, 6 ml). The suspension is subjected to ultrasonic treatment (9 KHz, 200 W, for 5 minutes) or to freezing - thawing [repeating three times freezing at -50°C (with acetone-dry ice) and thawing at 37°C] to fracture the cells, and the cell residue is removed by centrifugation at low speed (3,000 r.p.m., 20 minutes). The solution thus obtained is layered on glycerol cusion (5 %, 40 %) and then centrifuged at 35,000 r.p.m. for one hour. The pellets (0.5-1 ml) thus obtained are suspended in PBS (1-2 ml) and are treated with DNase (10 μg/ml) and RNase (0.3 mg/ml) at 37°C for one hour, and to the reaction mixture is added 1/5 volume of 5 x STEP [a mixture of 0.5 % SDS (sodium dodecyl-sulfate), 50 mM Tris-HCl (pH 7.5), 0.4 M EDTA, and 0.1 % proteinase K], and the mixture is reacted at 50°C for 30 minutes. The resulting solution is extracted with an equi-volume of phenol, phenol-chloroform (1 : 1) and chloroform in this order to give an aqueous layer containing DNA.

The aqueous layer is dialyzed against TE buffer (20 mM Tris-HCl, 1 mM EDTA, pH 7.5), and thereto is added cold ethanol to precipitate the DNA. The DNA is separated by filtration, dried in vacuum, and then dissolved in an aqueous cesium chloride [Rf 1.3885, incorporated with ethidium bromide (0.04 %) and lauroyl sarcosinate (0.4 %), 5 ml]. The mixture is centrifuged at 4,000 r.p.m. for 72 hours to form a band of HSV-1 DNA. The band is recovered and is washed with isopropyl alcohol to remove ethidium bromide, and then dialyzed against TE buffer. Cold ethanol is added thereto to precipitate HSV-1 DNA.

(ii) Cloning of BamHI-cleaved G fragment of HSV-1 DNA:

The HSV-1 DNA obtained above (about 100 μg) is treated with a restriction enzyme BamHI in a mixture (0.75 ml) of 73 mM Tris-HCl (pH 8.0), 7 mM MgCl₂, 100 mM NaCl and 2 mM 2-mercaptoethanol at 37°C for 6 hours, and then each fragment is separated by 0.7 % agarose electrophoresis to cut out a gel corresponding to G fragment (0.345-0.399 map units), from which the G fragment is electrophoretically recovered.

E. coli pBR322 plasmid (1/10 mole) obtained by cleavage with a restriction enzyme BamHI is reacted with the above G fragment (about 2 μg) in a mixture of 50 mM Tris-HCl (pH 7.9), 10 mM MgCl₂, 20 mM dithiothreitol and 1 mM ATP by using T₄ DNA ligase at 16°C for about 16 hours.

The above reaction mixture is added to a liquid of E. coli (0.1 ml) which is prepared by treating a

6

culture broth of E. coli $\chi$1776 (cf. Curtiss, R. III, "Molecular Cloning of Recombinant DNA" ed. Scott, W. A. and Werner, R., page 99, Academic Press, 1977) by the procedure as described in Norgard, M. V., Gene, 3 , 279 (1978), and the mixture is mixed well and allowed to stand at 0°C for 45 minutes. The mixture is applied onto an agar plate containing ampicillin (100 $\mu$g/ml) and then incubated at 37°C overnight. The resulting colonies are applied onto both an agar plate containing ampicillin (100 $\mu$g/ml) and an agar plate containing tetracycline (100 $\mu$g/ml), and are incubated likewise. The colonies which grow only on the agar plate containing ampicillin are selected. pBR322 has an ampicillin-resistant gene and a tetracycline-resistant gene, but when it is inserted with HSV-1 DNA fragment at the BamHI site of the tetracycline-resistant gene, it loses the tetracycline-resistance. Accordingly, the selected colonies contain a recombinant DNA of BamHI-G fragment of pBR322-HSV DNA.

From the colonies thus selected, a plasmid is prepared by the procedure as described by K. Matsubara (J. Virol., 16 , 479, 1975). The plasmid thus prepared is subjected to cleavage pattern analysis by treating with various restriction enzymes (e.g. BamHI, Bst EII, Kpn I, Sal I, Sst I, Xho I) to obtain a recombinant DNA of pBR322-BamHI-G fragment wherein BamHI-G fragment of HSV-1 DNA is inserted into pBR322 (hereinafter, referred to as "plasmid pG").

(iii) Base sequence of HSVgB gene:

The base sequence of the above plasmid pG containing HSVgB gene is determined as follows:

The plasmid pG (10 $\mu$l) is dissolved in a mixture (100 $\mu$l) of restriction enzymes Sma I and Sac I, and are reacted with Sma I (10 units) and Sac I (10 units) to give a fragment of Sma I - Sac I region containing the gB gene. This Sma I - Sac I fragment is treated with various restriction enzymes such as Sac I, Sac II, Sma I, Pst I, Sal I, Pvu II, Sau 3A and Nar I to obtain smaller fragments as shown in the accompanying Fig. 5 (by the symbols: ← → ), and these smaller segments are each inserted into a cloning vector M 13 mp 11 (sold by Pharmacia Japan) at the Sal I site thereof. According to dideoxy method [cf. "Proteins, Nucleic acids and Enzymes", Vol. 29, No. 4, 284-306 (1984), Item: "Method for the Determination of Base Sequence of DNA by Dideoxy Method"], the base sequence of the smaller fragments is determined. As a result, the Sma I - Sac I region has a base sequence and amino acid sequence of gB protein encoded thereby as shown in the accompanying Fig. 6. Thus, it is clear that the gB gene is composed of 903 amino acids.

(iv) Preparation of plasmid pGBX:

The plasmid pG (10 $\mu$g) obtained in the above (ii) is added to a mixture (100 $\mu$l) of 6 mM Tris-HCl (pH 7.5), 6 mM MgCl$_2$, 6 mM 2-mercaptoethanol and 150 mM NaCl, and thereto are added restriction enzymes BamHI (10 units) and Xho I (10 unites), and the mixture is reacted at 37°C for 2 hours. 3.5 kb fragment DNA is isolated from the reaction mixture by 1 % agarose gel electrophoresis in accordance with the method disclosed by Yasusuke Takagi, "Manual for Procedure of Genetic Engineering", pages 33-34.

The thus obtained 3.5 kb fragment DNA (100 ng) is reacted with E. coli plasmid pACYC 177 (which is cleaved with BamHI and Xho I) (10 ng) in a mixture (10 $\mu$l) of T$_4$ ligase (0.1 unit), 6.6 mM MgCl$_2$ and 10 mM dithiothreitol at 16°C for 8 hours. By using the above reaction mixture, E. coli $\chi$1776 is transformed in the same manner as described hereinbefore, and ampicillin resistant cells are selected from the resulting transformants. From the cells thus selected, a plasmid pGBX containing gB gene is prepared by the procedure as described by K. Matsubara (J. Virol., 16 , 479, 1975).

(v) Base sequence of Xho I - Sal I region (1.2 kb) of the plasmid pGBX:

The above-obtained plasmid pGBX (10 $\mu$g) is dissolved in a mixture (100 $\mu$l) of 6 mM Tris-HCl (pH 7.9), 6 mM MgCl$_2$ and 6 mM 2-mercaptoethanol, and thereto are added restriction enzymes Sal I (10 units) and Xho I (10 units), and the mixture is reacted at 37°C for 2 hours. The reaction mixture is subjected to 1 % agarose gel electrophoresis as above to isolate 1 .2 kb fragment.

The thus obtained 1 .2 kb fragment is inserted into a cloning vector M 13 mp 11 (sold by Pharmacia Japan) at the Sal I site thereof, and the base sequence thereof is determined by dideoxy method [cf. "Proteins, Nucleic acids and Enzymes", Vol. 29, No. 4, 294-305 (1984), Item: "Method for the Determination of Base Sequence of DNA by Dideoxy Method"]. As a result, it shows the base sequence as is shown in the accompanying Fig. 3. As is clear from the figure, the Xho I site is distant about 250 bp from the translation initiation codon ATG of the gB gene and further Apa I site is present at 14 bp - 19 bp upstream from the ATG.

(vi) Preparation of plasmid pGAB:

The above plasmid pGBX is treated in the following manner in order to convert only the Apa I site upstream of the translation initiation codon ATG of the gB gene into Xho I site.

Plasmid pGBX (2 $\mu$g), Apa I (0.1 unit) and a mixture (50 $\mu$l) of 6 mh Tris-HCl (pH 7.5), 6 mM NaCl, 6 mM MgCl$_2$ and 6 mM 2-mercaptoethanol are mixed and the mixture is reacted at 37°C for 5 minutes.

The reaction mixture is subjected to phenol extraction and ethanol precipitation. The resulting DNA is treated with $T_4$ DNA polymerase (0.1 unit) in a solution (50 $\mu$l) of 67 mM Tris-HCl (pH 8.6), 6.7 mM $MgCl_2$, 10 mM 2-mercaptoethanol, 16.7 mM $(NH_4)_2SO_4$ which contains 200 $\mu$M $\alpha$ATP, $\alpha$CTP, $\alpha$GTP and $\alpha$TTP at 37°C for 30 minutes. The reaction mixture is subjected to phenol extraction and ethanol precipitation. The resulting DNA (1 pmole) is subjected to linking reaction with Xho I linker by using $T_4$ ligase (0.1 unit) in a mixture (10 $\mu$l) of 66 mM Tris-HCl (pH 7.6), 6.6 mM $MgCl_2$, 10 mM dithiothreitol, 66 $\mu$M ATP and Xho I linker (1 pmole) at 16°C for 8 hours. E. coli $\chi$1776 is transformed with the reaction mixture obtained above in the same manner as described hereinbefore. A plenty of clones which grow on an ampicillin-containing plate are selected, and the plasmids are prepared from the selected cells in the same manner as described above. The plasmids are each treated with a restriction enzyme Xho I, and thereby there is selected the desired plasmid pGAB by the patterns in electrophoresis. In the plasmid pGAB, only Apa I site upstream of the translation initiation codon ATG of the gB gene is converted into Xho I site.

(vii) Preparation of Sac I fragment wherein the tail region of the gB gene is removed:

The plasmid pG (10 $\mu$g) obtained in the above (ii) is treated with Sac I (10 units) in a mixture (100 $\mu$l) of 6 mM Tris-HCl (pH 7.5), 6 mM $MgCl_2$, 6 mM 2-mercaptoethanol and 150 mM NaCl at 37°C for 2 hours. The reaction mixture is subjected to 1 % agarose gel electrophoresis and the desired DNA fragment (Sac I fragment) (2.7 kb, containing about 93 % of the gB gene) is extracted and isolated in the same manner as described hereinbefore.

## (2) Preparation of shuttle vectors:

### (A) shuttle vector pONY1:

Shuttle vector pAM 82 as prepared in the same manner as described in Japanese Patent First Publication No. 31799/1984 is treated as follows in order to convert the Puv II site into BamHI site.

A fragment (2 $\mu$g) prepared by cleaving the plasmid pAM 82 with Xho I is reacted with $T_4$ DNA polymerase (0.1 unit) in a mixture (50 $\mu$l) of 67 mM Tris-HCl (pH 8.6), 6.7 mM $MgCl_2$, 10 mM 2-mercaptoethanol, 6.7 $\mu$M EDTA and 16.7 mM $(NH_4)_2SO_4$ which contains 200 $\mu$M $\alpha$ATP, $\alpha$CTP, $\alpha$GTP and $\alpha$TTP at 37°C for 30 minutes. The reaction mixture is subjected to phenol extraction and ethanol precipitation. The resulting DNA is reacted with BamHI linker in a molar ratio of 1 : 10 by using $T_4$ ligase at 16°C for 8 hours, and E. coli $\chi$1776 is transformed with the reaction mixture obtained above in the same manner as described hereinbefore. The ampicillin-resistant cells thus obtained are incubated, and from the cells thus obtained, there is isolated a plasmid pONY1 in the same manner as described hereinbefore. In said plasmid pONY1, the Pvu II site of pAM 82 is converted into BamHI site.

### (B) Shuttle vector pAM 82 (Sac):

Shuttle vector pAM 82 as prepared in the same manner as described in Japanese Patent First Publication No. 31799/1984 is treated as follows in order to convert the Xho I site into Sac I site.

A fragment (2 $\mu$g) prepared by cleaving the plasmid pAM 82 with Xho I is reacted with $T_4$ DNA polymerase (0.1 unit) in a mixture (50 $\mu$l) of 67 mM Tris-HCl (pH 8.6), 6.7 mM $MgCl_2$, 10 mM 2-mercaptoethanol, 6.7 $\mu$M EDTA and 16.7 mM $(NH_4)_2SO_4$ which contains 200 $\mu$M $\alpha$ATP, $\alpha$CTP, $\alpha$GTP and $\alpha$TTP at 37°C for 30 minutes. The reaction mixture is subjected to phenol extraction and ethanol precipitation. The resulting DNA is reacted with Sac I linker in a molar ratio of 1 : 10 by using $T_4$ ligase at 16°C for 8 hours, and E. coli $\chi$1776 is transformed with the reaction mixture obtained above in the same manner as described hereinbefore. The ampicillin-resistant cells thus obtained are incubated, and from the cells thus obtained, there is isolated a plasmid pAM 82 (Sac) in the same manner as described hereinbefore. In said plasmid pAM 82 (Sac), Xho I site of pAM 82 is converted into Sac I site.

## (3) Preparation of HSVgB expression plasmid:

(A) The plasmid pGAB (10 $\mu$g) containing whole of HSVgB gene as prepared in the above (1), (iv) is reacted with BamHI (10 units) and Xho I (10 units) in a mixture (100 $\mu$l) of 6 mM Tris-HCl (pH 7.5), 6 mM $MgCl_2$, 6 mM 2-mercaptoethanol and 150 mM NaCl at 37°C for 2 hours. From the reaction mixture there is isolated a DNA fragment (3.3 kb) containing HSVgB gene by 1 % agarose electrophoresis.

Separately, the shuttle vector pONY1 (10 $\mu$g) obtained in the above (2), (A) is treated with restriction enzymes BamHI and Xho I in the same manner as described hereinbefore, and from the reaction mixture there is isolated a DNA fragment (10 kb) containing acid phosphatase promoter by 1 % agarose electrophoresis.

The 3.3 kb fragement (10 ng) and the 10 kb fragment (10 ng) obtained above are reacted with T₄ ligase (0.1 unit) in a mixture (10 μl) of 66 mM Tris-HCl (pH 7.6), 6.6 mM MgCl₂, 10 mM dithiothreitol and 66 μM ATP at 16°C for 8 hours. E. coli χ1776 is transformed with the reaction mixture obtained above in the same manner as described hereinbefore. The ampicillin-resistant cells thus obtained are incubated, and from the cells thus obtained, there is isolated a recombinant plasmid pONYGB wherein HSVgB gene is inserted at the downstream of the acid phosphatase promoter in the same manner as described hereinbefore.

(B) The plasmid pAM 82 (Sac) (1 μg) as prepared in the above (2), (B) is reacted with Sac I (10 units) in a mixture (100 μl) of 6 mM Tris-HCl (pH 7.5), 6 mM MgCl₂, 6 mM 2-mercaptoethanol and 150 mM NaCl at 37°C for 2 hours. The reaction mixture is subjected to phenol extraction and ethanol precipitation, and therefrom there is isolated a DNA fragment (3.3 kb) containing HSVgB gene in the same manner as described above.

The fragment (10 ng) obtained above and the 2.7 kb Sac I fragment (100 ng) obtained in the above (1), (vii) are reacted with T₄ ligase (0.1 unit) in a mixture (10 μl) of 66 mM Tris-HCl (pH 7.6), 6.6 mM MgCl₂, 10 mM dithiothreitol and 66 μM ATP at 16°C for 8 hours. E. coli χ1776 is transformed with the reaction mixture obtained above in the same manner as described hereinbefore. The ampicillin-resistant cells thus obtained are incubated, and from the cells thus obtained, there is isolated a recombinant plasmid pAMGB1 in the same manner as described above. In said recombinant plasmid pAMGB1, HSVgB gene losing the tail region (i.e. HSVgB gene having about 90 % of the whole gB gene, 816 amino acids) is inserted at the downstream of the acid phosphatase promoter and at the downstream therefrom a vector-origin gene (four amino acids) is inserted.

(4) Transformation of yeast:

(A) The starting yeast is Saccharomyces cerevisiae AH 22 [a, leu 2, his 4, can 1 (Cir⁺)], which has been deposited at Fermentation Research Institute, Agency of Industrial Science and Technology, Japan under Budapest Treaty as "FERM BP-312". The starting yeast is inoculated in YPD medium (100 ml) consisting of 2 % polypeptone, 1 % yeast extract and 2 % glucose, and the mixture is incubated at 30°C overnight, and thereafter, the cells are collected by centrifugation. The cells thus collected are washed with sterilized water (20 ml), suspended in a solution (5 ml) of 1.2 M sorbitol and Zymolyase-60,000 (manufactured by Seikagaku Kogyo K.K., Japan, 100 μg/ml), and the suspension is allowed to stand at 30°C for 30 minutes to give spheroplast. The spheroplast thus prepared is washed with 1.2 M sorbitol solution three times, and then suspended in a solution (0.6 ml) of 2 M sorbitol, 10 mM CaCl₂ and 10 mM Tris-HCl (pH 7.5). The suspension thus prepared is divided into a small test tube in a volume of 60 μl. To the suspension is added the solution of the recombinant plasmid pONYGB (10 μg) as prepared in the above (3), (A). After mixing well, 0.1 M CaCl₂ (3 μl) is added thereto in a final concentration of 10 mM CaCl₂, and the mixture is allowed to stand at room temperature for 5 to 10 minutes. To the resulting mixture is added each 1 ml of a solution of 20 % polyethylene glycol 4,000, 10 mM CaCl₂ and 10 mM Tris-HCl (pH 7.5), and the mixture is allowed to stand at room temperature for about 20 minutes. The resulting mixture (each 0.2 ml) is added to a medium (10 ml) consisting of 22 % sorbitol, 2 % glucose, 0.7 % yeast nitrogen base amino acid, 2 % YPD, 20 μg/ml histidine and 3 % agar, which is kept at a constant temperature of 45°C. After gently mixing, the mixture is added in a layer onto a plate of minimal medium containing 1.2 M sorbitol which is previously prepared and consists of 0.7 % yeast nitrogen base amino acid, 2 % glucose, 20 μg/ml histidine and 2 % agar and is set thereon. The plate is incubated at 30°C to give a colonie of a leucine-non-requiring yeast. The colonie is incubated in a BurkHorlder minimal medium supplemented with histidine (20 μg/ml) [cf. Tohe, A. et al; J. Bacterol., 113, 727-738, 1973] to give the desired transformed yeast: Saccharomyces cerevisiae YGB.

(B) By using Saccharomyces cerevisiae AH 22 [a, leu 2, his 4, can 1 (Cir⁺)] (FERM BP-312) as the starting yeast, it is inoculated in YPD medium (100 ml) consisting of 2 % polypeptone, 1 % yeast extract and 2 % glucose, and the mixture is incubated at 30°C overnight, and thereafter, the cells are collected by centrifugation. The cells thus collected are washed with sterilized water (20 ml), suspended in a solution (5 ml) of 1.2 M sorbitol and Zymolyase-60,000 (manufactured by Seikagaku Kogyo K.K., Japan, 100 μg/ml), and the suspension is allowed to stand at 30°C for 30 minutes to give spheroplast. The spheroplast thus prepared is washed with 1.2 M sorbitol solution three times, and then suspended in a solution (0.6 ml) of 2 M sorbitol, 10 mM CaCl₂ and 10 mM Tris-HCl (pH 7.5). The suspension thus prepared is divided into a small test tube in a volume of 60 μl. To the suspension is added the solution of the recombinant plasmid pAMGB1 (10 μg) as prepared in the above (3), (B). After mixing well, 0.1 M CaCl₂ (3 μl) is added thereto in a final concentration of 10 mM CaCl₂, and the mixture is allowed to stand at room temperature for 5 to 10 minutes. To the resulting mixture is added each 1 ml of a solution of 20 % polyethylene glycol 4,000, 10 mM CaCl₂ and 10 mM Tris-HCl (pH 7.5), and the mixture is allowed to stand at room temperature for about 20 minutes. The resulting mixture (each 0.2 ml) is added to a medium (10 ml) consisting of 22 % sorbitol, 2 % glucose, 0.7 % yeast nitrogen base amino acid, 2 % YPD, 20 μg/ml histidine and 3 % agar, which is kept at a constant temperature of 45°C. After gently mixing, the mixture is added in a layer onto a plate of minimal medium containing 1.2 M sorbitol which is previously prepared and consists of 0.7% yeast nitrogen base amino acid, 2 % glucose, 20 μg/ml histidine and 2 % agar and is set thereon. The plate is incubated at 30°C to give a colonie of a leucine-non-requiring yeast. The colonie is incubated in a BurkHorlder minimal medium supplemented with histidine (20 μg/ml) [cf. Tohe, A. et al; J. Bacterol., 113 , 727-738, 1973] to give the desired transformed yeast.

(5) Production of HSVgB by the transformed yeast:

(A) The colonie of the transformed yeast obtained in the above (4), (A) is applied onto an agar plate of BurkHolder minimal medium supplemented with histidine (20 μg/ml) and incubated at 30°C to form a colonie (in order to confirm the transformant requiring no leucine). The resulting cells are separated from the colonie, inoculated in BurkHolder minimal medium (10 ml) supplemented with histidine (20 μg/ml) and incubated at 30°C. After about 24 hours, the cells in logarithmic growth phase are collected by centrifugation and are suspended in a minimal medium (10 ml) containing no phosphoric acid (which is prepared by replacing KH₂PO₄ in BurkHolder minimal medium with KCl, followed by supplementing 20 μg/ml histidine) in a cell concentration of about 4 x 10⁶ cells/ml. After incubating at 30°C for about 24 hours, the culture broth is centrifuged at 4,000 r.p.m. for 10 minutes to collect the cells. The cells thus separated are suspended in a solution (3 ml) of 1.2 M sorbitol, 50 mM phosphate buffer (pH 7.2), 14 mM 2-mercaptoethanol and 100 μg/ml Zymolyase-60,000 (manufactured by Seikagaku Kogyo K.K., Japan), and the mixture is gently shaken at 30°C for 30 minutes to give spheroplast. The spheroplast is collected by centrifugation and is well suspended in 50 mM phosphate buffer (pH 7.2, 1 ml) containing 1 % Tritone χ-100, and the mixture is vigorously stirred with glass beads to fracture the cells. The resulting fractured mixture is centrifuged at 5,000 r.p.m. for 10 minutes, and the resulting supernatant is taken as the yeast-lysed solution.

As to the supernatant, the gB antigen activity was measured by an enzyme immunoassay. The results are shown in Table 1.

Besides, the fractured solution obtained above (each 1 ml) was subcutaneously administered to guinea pigs (5 animals) four times in each other week. As a result, there was observed neutralizing antibody in all guinea pigs.

## Table 1

| Run No. | 1 | 2 | 3 | 4 | 5 | 6 | 7 | 8 |
|---|---|---|---|---|---|---|---|---|
| gB activity (OD₄₉₂) | 0.10 | 0.06 | 0.12 | 0.12 | 0.27 | 0.21 | 0.01 | 0.45 |

[Note]:   Run Nos. 1-6:   Yeast clone (TGB) which were transformed by plasmid pONYGB

Run No. 7:   Yeast having no plasmid pONYGB

Run No. 8:   HSV-origin gB

(B) In the same manner as described in the above (A) except that the colonie of transformed yeast obtained in the above (4), (B) is used instead of the colonie of transformed yeast obtained in (4), (A), the cells of the transformed yeast are collected, fractured and centrifuged likewise.

As to the supernatant, the gB antigen activity was measured by an enzyme immunoassay, likewise. The results are shown in Table 2.

Besides, the fractured solution obtained above (each 1 ml) was subcutaneously administered to guinea pigs (5 animals) four times in each other week. As a result, there was observed neutralizing antibody in all guinea pigs.

## Table 2

| Run No. | 11 | 12 | 13 | 14 | 15 | 16 | 17 | 18 |
|---|---|---|---|---|---|---|---|---|
| gB activity $(OD_{492})$ | 0.20 | 0.25 | 0.30 | 0.21 | 0.25 | 0.22 | 0.01 | 0.45 |

[Note]: Run Nos. 11-16: Yeast clone (TGB) which were transformed by plasmid pAMGB1

Run No. 17: Yeast having no plasmid pAMGB1

Run No. 18: HSV-origin gB

## Claims

1. A recombinant plasmid which comprises a plasmid vector containing
    (a) a yeast DNA sequence;
    (b) an Escherichia coli DNA sequence;
    (c) the expression control region of the repressible yeast acid phosphatase gene; and
    (d) recombined to and under the control of (c) a DNA fragment in which part of the 3' region is removed and the resulting DNA fragment comprises about 90% of the whole herpes simplex virus (HSV) gB gene encoding 903 amino acids.

2. The recombinant plasmid according to claim 1, wherein said expression control region (c) is derived from a gene of a polypeptide of 60,000 dalton (P60) which constitutes the phosphatase, a part or whole of the structural gene of the phosphatase, wherein a region upstream therefrom in the range of from +1 (ATG) to -100 bp is optionally deleted.

3. The recombinant plasmid according to claim 1 or 2, wherein the yeast DNA sequence (a) contains ars 1, the 2μ ori and a selective marker gene for a transformed yeast which is a leucine-producing gene, a histidine-producing gene, a tryptophane-producing gene, a uracil-producing gene, or an adenine-producing gene, or a combination of two or more thereof.

4. The recombinant plasmid according to any one of claims 1 to 3, wherein the E. coli DNA sequence (b) contains a DNA sequence which is necessary for replication of the plasmid in cells of E. coli and a selective marker gene for a transformed E. coli which is an ampicillin-resistance gene, a kanamycin-resistance gene, a tetracycline-resistance gene, or a chloramphenicol-resistance gene, or a combination of two or more thereof.

5. The recombinant plasmid according to claim 4, wherein the E. coli DNA sequence (b) is the E. coli plasmid pBR322 that contains an ampicillin-resistance gene and a tetracycline-resistance gene.

6. The recombinant plasmid according to any one of claims 1 to 5, wherein the DNA fragment (d) does not contain the 3'-region of the gB gene.

7. The recombinant plasmid according to any one of claims 1 to 6, wherein the DNA fragment (d) without the 3'-region of the gB gene is an Apa I/Sac I fragment of the HSV gB gene.

8. The recombinant plasmid according to any one of claims 1 to 6, wherein the DNA fragment (d) without the 3'-region of the gB gene is a fragment containing about 90% of the gB gene which is obtainable by treating the gB gene with the restriction enzyme Sac I.

9. A transformed yeast which is prepared by transforming a yeast with the recombinant plasmid according to any one of claims 1 to 8.

10. The transformed yeast according to claim 9, wherein the starting yeast is the leucine-requiring mutant strain Saccharomyces cerevisiae AH 22 [a, leu 2, his 4, Can 1 (Cir$^+$)] or Saccharomyces cerevisiae AH 22 pho 80.

11. A method for the production of the herpes simplex virus gB protein, which comprises culturing the transformed yeast of claim 9 or 10 in order to produce the herpes simplex virus gB protein and collecting the protein.

12. The method according to claim 11 , wherein the culturing of the transformed yeast is carried out under conditions where the acid phosphatase promoter is not repressed.

13. A method for the production of a recombinant plasmid according to any one of claims 1 to 8 , which comprises inserting the herpes simplex virus gB gene into a shuttle vector downstream of the expression control region (c) and under the control of the phosphatase promoter contained in (c).

14. A method for the production of a transformed yeast, which comprises constructing a recombinant plasmid according to any one of claims 1 to 8 by inserting the herpes simplex virus gB gene into a shuttle vector down-stream of the expression control region (c) and under the control of the phosphatase promoter contained in (c) and transforming a yeast with the resulting recombinant plasmid according to any one of claims 1 to 8 .

**Revendications**

1. Plasmide recombinant qui comprend un vecteur plasmidique contenant
    (a) une séquence d'ADN de levure,
    (b) une séquence d'ADN d'Escherichia coli,
    (c) la région de commande de l'expression du gène de la phosphatase acide de levure répressible et
    (d) recombiné à et sous la commande de (c), un fragment d'ADN dans lequel une partie de la région 3' est enlevée et le fragment d'ADN résultant comprend environ 90% du gène gB de l'Herpes simplex virus (HSV) complet, encodant 903 aminoacides.

2. Plasmide recombinant suivant la revendication 1, caractérisé en ce que la région de commande de l'expression (c) provient d'un gène d'un polypeptide de 60.000 daltons (P60) qui constitue la phosphatase, une partie ou la totalité du gène structurel de la phosphatase, où une région en amont de celle-ci dans la gamme de +1 (ATG) à -100 pb est éventuellement supprimée.

3. Plasmide recombinant suivant la revendication 1 ou 2, caractérisé en ce que la séquence d'ADN de levure (a) contient ars 1, le 2μ ori et un gène marqueur sélectif pour une levure transformée, qui est un gène producteur de leucine, un gène producteur d'histidine, un gène producteur de tryptophane, un gène producteur d'uracile, un gène producteur d'adénine, ou une combinaison de deux ou de plus de deux d'entre eux.

4. Plasmide recombinant suivant l'une quelconque des revendications 1 à 3, caractérisé en ce que la séquence d'ADN Escherichia coli (b) contient une séquence d'ADN qui est nécessaire à la réplication du plasmide dans des cellules d'Escherichia coli et un gène marqueur sélectif pour un Escherichia coli transformé, qui est un gène de résistance à l'ampicilline, un gène de résistance à la kanamycine, un gène de résistance à la tétracycline, ou un gène de résistance au chloramphénicol ou une combinaison de deux ou de plus de deux d'entre eux.

5. Plasmide recombinant suivant la revendication 4, caractérisé en ce que la séquence d'ADN d'Escherichia coli (b) est le plasmide d'Escherichia coli pBR322 qui contient un gène de résistance à l'ampicilline et un gène de résistance à la tétracycline.

6. Plasmide recombinant suivant l'une quelconque des revendications 1 à 5, caractérisé en ce que le fragment d'ADN (d) ne contient pas la région 3' du gène gB.

7. Plasmide recombinant suivant l'une quelconque des revendications 1 à 6, caractérisé en ce que le fragment d'ADN (d) sans la région 3' du gène gB est un fragment d'Apa I/Sac I du gène gB de l'HSV.

8. Plasmide recombinant suivant l'une quelconque des revendications 1 à 6, caractérisé en ce que le fragment d'ADN (d) sans la région 3' du gène gB est un fragment contenant environ 90% du gène gB que l'on peut obtenir par le traitement du gène gB par l'enzyme de restriction SaC I.

9. Levure transformée, caractérisée en ce qu'on la prépare par la transformation d'une levure par le plasmide recombinant suivant l'une quelconque des revendications 1 à 8.

10. Levure transformée suivant la revendication 9, caractérisée en ce que la levure de départ est la souche mutante exigeant de la leucine, Saccharomyces cerevisiae AH 22 [a, leu 2, his 4, Can 1 (Cir$^+$)] ou Saccharomyces cerevisiae AH 22 pho 80.

11. Procédé de production de la protéine gB de l'Herpes simplex virus, caractérisé en ce que l'on procède à la culture de la levure transformée de la revendication 9 ou 10 en vue de produire la protéine gB de l'Herpes simplex virus et on recueille la protéine.

12. Procédé suivant la revendication 11, caractérisé en ce que l'on procède à la culture de la levure transformée dans des conditions telles que le promoteur de phosphatase acide ne soit pas réprimé.

13. Procédé de production d'un plasmide recombinant suivant l'une quelconque des revendications 1 à 8, caractérisé en ce que l'on insère le gène gB de l'Herpes simplex virus dans un vecteur-vanne en aval de la région de commande de l'expression (c) et sous la commande du promoteur de phosphatase contenu dans (c).

14. Procédé de production d'une levure transformée, caractérisé en ce qu'il comprend la construction d'un plasmide recombinant suivant l'une quelconque des revendications 1 à 8 par l'insertion du gène gB de l'Herpes simplex virus dans un vecteur-vanne en aval de la région de commande de l'expression (c) et sous la commande du promoteur de phosphatase contenu dans (c) et la transformation d'une levure à l'aide du plasmide recombinant résultant suivant l'une quelconque des revendications 1 à 8.

## Ansprüche

1. Rekombinantes Plasmid, das einen Plasmidvektor umfaßt, der
   (a) eine DNA-Sequenz aus Hefe,
   (b) eine DNA-Sequenz aus Escherichia coli ,
   (c) die Expressionskontrollregion des reprimierbaren Saure-Phosphatase-Genes aus Hefe, und
   (d) rekombiniert mit und unter der Kontrolle von (c), ein DNA-Fragment, von dem ein Teil des 3'-Bereiches entfernt ist und das resultierende DNA-Fragment ungefähr 90 % des gesamten Herpes simplex-Virus (HSV) gB-Genes, das 903 Aminosäuren codiert, umfaßt, enthält.

2. Rekombinantes Plasmid nach Anspruch 1, in dem die Expressionskontrollregion (c) aus einem Gen für ein Polypeptid von 60 000 Dalton (P60) stammt, das die Phosphatase, einen Teil oder das ganze Strukturgen der Phosphatase darstellt, wobei eine Region aufwärts davon im Bereich von +1 (ATG) bis -100 bp gegebenenfalls entfernt ist.

3. Rekombinantes Plasmid nach Anspruch 1 oder 2, in dem die Hefe-DNA-Sequenz (a) ars 1, den 2μ Ursprung und ein selektives Markergen für eine transformierte Hefe, das ein Leucin-herstellendes Gen, ein Histidin-herstellendes Gen, ein Tryptophan-herstellendes Gen, ein Uracil-herstellendes Gen oder ein Adenin-herstellendes Gen, oder eine Kombination von zwei oder mehreren davon ist, enthält.

4. Rekombinantes Plasmid nach einem der Ansprüche 1 bis 3, in dem die E. coli DNA Sequenz (b) eine DNA-Sequenz, die für die Replikation des Plasmides in Zellen von E. coli nötig ist, und ein selektives Markergen für einen transformierten E. coli , das ein Ampicillin-Resistenzgen, ein Kanamycin-Resistenzgen, ein Tetracyclin-Resistenzgen oder ein Chloramphenicol-Resistenzgen, oder eine Kombination von zwei oder mehreren davon ist, enthält.

5. Rekombinantes Plasmid nach Anspruch 4, in dem die E. coli DNA-Sequenz (b) das E. coli Plasmid pBR322 ist, das ein Ampicillin-Resistenzgen und ein Tetracyclin-Resistenzgen enthält.

6. Rekombinantes Plasmid nach einem der Ansprüche 1 bis 5, in dem das DNA-Fragment (d) keine 3'-Region des gB-Genes enthält.

7. Rekombinantes Plasmid nach einem der Ansprüche 1 bis 6, in dem das DNA-Fragment (d) ohne die 3'-Region des gB-Genes ein Apa I/Sac I-Fragment des HSV gB-Genes ist.

8. Rekombinantes Plasmid nach einem der Ansprüche 1 bis 6, in dem das DNA-Fragment (d) ohne die 3'-Region des gB-Genes ein etwa 90 % des gB-Genes enthaltendes Fragment ist, das durch Behandlung des gB-Genes mit dem Restriktionsenzym Sac I erhältlich ist.

9. Transformierte Hefe, die durch Transformation einer Hefe mit dem rekombinanten Plasmid nach einem der Ansprüche 1 bis 8 hergestellt wird.

10. Transformierte Hefe nach Anspruch 9, wobei die Ausgangshefe die Leucin-benötigende Mutante, Stamm Saccharomyces cerevisiae AH 22 [a, leu 2, his 4, Can 1 (Cir$^+$)] oder Saccharomyces cerevisiae AH 22 pho 80, ist.

11. Verfahren zur Herstellung von Herpes simplex Virus gB-Protein, das die Kultivierung der transformierten Hefe nach Anspruch 9 oder 10 zur Erzeugung des Herpes simplex-Virus gB-Proteins und die Gewinnung des Proteins umfaßt.

12. Verfahren nach Anspruch 11, in dem die Kultivierung der transformierten Hefe unter Bedingungen durchgeführt wird, die den Saure-Phosphatase-Promotor nicht reprimieren.

13. Verfahren zur Herstellung eines rekombinanten Plasmids nach einem der Ansprüche 1 bis 8, das die Insertion des Herpes simplex Virus gB-Genes in einen "Shuttlevektor", abwärts der Expressionskontrollregion (c) und unter der Kontrolle des in (c) enthaltenen Phosphatase-Promotors, umfaßt.

14. Verfahren zur Herstellung einer transformierten Hefe, das die Konstruktion eines rekombinanten Plasmides nach einem der Ansprüche 1 bis 8 durch Insertion des Herpes simplex Virus gB-Genes in einen "Shuttlevektor", abwärts der Expressionskontrollregion (c) und unter der Kontrolle des in (c) enthaltenen Phosphatase-Promotors, und die Transformation einer Hefe mit dem resultierenden rekombinanten Plasmid nach einem der Ansprüche 1 bis 8 umfaßt.

Fig. 1

Fig. 2

Fig. 3

EP 0 170 169 B1

CTCGAG

Xho I

301 – TTGCGCCGCCCGGACTGCAGCCGCCCGACCTCCGAAGGTCGTTACCGTTACCCGCCCGGCGTATATCTCACGTACGACTCCGACTGTCCGCTGGTGGCCA

401 – TCGTCGAGAGCGCCCCCGACGGCTGTATCGGCCCCCGGTCGGTCGTGGTCTACGACGCCGACGTTTTCTCGATCCTCTACTCGGTCCTCCAGCACCTCGC

501 – CCCCAGGCTACCTGACGGGGGGCACGAC GGGCCC CCGTAGTCCCGCC ATG CAC CAG GGC GCC CCC TCG TGG GGG CGC CGG TGG TTC

Apa I          met his gln gly ala pro ser trp gly arg arg trp phe – 13

587 – GTC GTA TGG GCG CTC TTG GGG TTG ACG CTG GGG GTC CTG GTG GCG TCG GCG GCT CCG AGT TCC CCC GGC ACG CCT

val val trp ala leu leu gly leu thr leu gly val leu val ala ser ala ala pro ser ser pro gly thr pro – 38

Fig. 4

Fig. 7

Fig. 5

HSV  DNA

EP 0 170 169 B1

Fig. 6 (A)

GAGCTCGAGTTGCGCCGCCCGGACTGCAGCCGCCCGACCTCCGAAGGTCGTTACCGTTACCCGCCCGGCGTA

TATCTCACGTACGACTCCGACTGTCCGCTGGTGGCCATCGTCGAGAGCGCCCCCGACGGCTGTATCGGCCCC

CGGTCGGTCGTGGTCTACGACGCCGACGTTTTCTCGATCCTCTACTCGGTCCTCCAGCACCTCGCCCCCAGG

CTACCTGACGGGGGGGCACGACGGGCCCCCGTAGTCCCGCC ATG CAC CAG GGC GCC CCC TCG TGG GGG CGC
                                                   Met-His-Gln-Gly-Ala-Pro-Ser-Trp-Gly-Arg-

CGG TGG TTC GTC GTA TGG GCG CTC TTG GGG TTG ACG CTG GGG GTC CTG GTG GCG TCG GCG
Arg-Trp-Phe-Val-Val-Trp-Ala-Leu-Leu-Gly-Leu-Thr-Leu-Gly-Val-Leu-Val-Ala-Ser-Ala-

GCT CCG AGT TCC CCC GGC ACG CCT GGG GTC GCG CGC GAC CCA GGC GGC GAA CGG GGG CCC
Ala-Pro-Ser-Ser-Pro-Gly-Thr-Pro-Gly-Val-Ala-Arg-Asp-Pro-Gly-Gly-Glu-Arg-Gly-Pro-

TGC CAC TCC GGC GCC GCC GCC CTT GGC GCC GCC CCA ACG GGG GAC CCG AAA CCG AAG AAG
Cys-His-Ser-Gly-Ala-Ala-Ala-Leu-Gly-Ala-Ala-Pro-Thr-Gly-Asp-Pro-Lys-Pro-Lys-Lys-

AAC AAA AAA CCG AAA AAC CCA ACG CCA CCA CGC CCC GCC GGC GAC AAC GCG ACC GTC GCC
Asn-Lys-Lys-Pro-Lys-Asn-Pro-Thr-Pro-Pro-Arg-Pro-Ala-Gly-Asp-Asn-Ala-Thr-Val-Ala-

GCG GGC CAC GCC ACC CTG CGC GAG CAC CTG CGG GAC ATC AAG GCG GAG AAC ACC GAT GCA
Ala-Gly-His-Ala-Thr-Leu-Arg-Glu-His-Leu-Arg-Asp-Ile-Lys-Ala-Glu-Asn-Thr-Asp-Ala-

AAC TTT TAC GTG TGC CCA CCC CCC ACG. GGC GCC ACG GTG GTG CAG TTC GAG CAG CCG CGC
Asn-Phe-Tyr-Val-Cys-Pro-Pro-Pro-Thr-Gly-Ala-Thr-Val-Val-Gln-Phe-Glu-Gln-Pro-Arg-

CGC TGC CCG ACC CGG CCC GAG GGT CAG AAC TAC ACG GAG GGC ATC GCG GTG GTC TTC AAG
Arg-Cys-Pro-Thr-Arg-Pro-Glu-Gly-Gln-Asn-Tyr-Thr-Glu-Gly-Ile-Ala-Val-Val-Phe-Lys-

GAG AAC ATC GCC CCG TAC AAG TTC AAG GCC ACC ATG TAC TAC AAA GAC GTC ACC GTT TCG
Glu-Asn-Ile-Ala-Pro-Tyr-Lys-Phe-Lys-Ala-Thr-Met-Tyr-Tyr-Lys-Asp-Val-Thr-Val-Ser-

EP 0 170 169 B1

Fig. 6 (B)

```
CAG GTG TGG TTC GGC CAC CGC TAC TCC CAG TTT ATG GGG ATC TTT GAG GAC CGC GCC CCC
Gln-Val-Trp-Phe-Gly-His-Arg-Tyr-Ser-Gln-Phe-Met-Gly-Ile-Phe-Glu-Asp-Arg-Ala-Pro-

GTC CCC TTC GAG GAG GTG ATC GAC AAG ATC AAC GCC AAG GGG GTC TGT CGG TCC ACG GCC
Val-Pro-Phe-Glu-Glu-Val-Ile-Asp-Lys-Ile-Asn-Ala-Lys-Gly-Val-Cys-Arg-Ser-Thr-Ala-

AAG TAC GTG CGC AAC AAC CTG GAG ACC ACC GCG TTT CAC CGG GAC GAC CAC GAG ACC GAC
Lys-Tyr-Val-Arg-Asn-Asn-Leu-Glu-Thr-Thr-Ala-Phe-His-Arg-Asp-Asp-His-Glu-Thr-Asp-

ATG GAG CTG AAA CCG GCC AAC GCC GCG ACC CGC ACG AGC CGG GGC TGG CAC ACC ACC GAC
Met-Glu-Leu-Lys-Pro-Ala-Asn-Ala-Ala-Thr-Arg-Thr-Ser-Arg-Gly-Trp-His-Thr-Thr-Asp

CTC AAG TAC AAC CCC TCG CGG GTG GAG GCG TTC CAC CGG TAC GGG ACG ACG GTA AAC TGC
Leu-Lys-Tyr-Asn-Pro-Ser-Arg-Val-Glu-Ala-Phe-His-Arg-Tyr-Gly-Thr-Thr-Val-Asn-Cys-

ATC GTC GAG GAG GTG GAC GCG CGC TCG GTG TAC CCG TAC GAC GAG TTT GTG CTG GCG ACT
Ile-Val-Glu-Glu-Val-Asp-Ala-Arg-Ser-Val-Tyr-Pro-Tyr-Asp-Glu-Phe-Val-Leu-Ala-Thr-

GGC GAC TTT GTG TAC ATG TCC CCG TTT TAC GGC TAC CGG GAG GGG TCG CAC ACC GAA CAC
Gly-Asp-Phe-Val-Tyr-Met-Ser-Pro-Phe-Tyr-Gly-Tyr-Arg-Glu-Gly-Ser-His-Thr-Glu-His-

ACC ACG TAC GCC GCC GAC CGC TTC AAG CAG GTC GAC GGC TTC TAC GCG CGC GAC CTC ACC
Thr-Thr-Tyr-Ala-Ala-Asp-Arg-Phe-Lys-Gln-Val-Asp-Gly-Phe-Tyr-Ala-Arg-Asp-Leu-Thr-

ACC AAG GCC CGG GCC ACG GCG CCG ACC ACC CGG AAC CTG CTC ACG ACC CCC AAG TTC ACC
Thr-Lys-Ala-Arg-Ala-Thr-Ala-Pro-Thr-Thr-Arg-Asn-Leu-Leu-Thr-Thr-Pro-Lys-Phe-Thr-

GTG GCC TGG GAC TGG GTG CCA AAG CGC CCG TCG GTC TGC ACC ATG ACC AAG TGG CAG GAA
Val-Ala-Trp-Asp-Trp-Val-Pro-Lys-Arg-Pro-Ser-Val-Cys-Thr-Met-Thr-Lys-Trp-Gln-Glu-

GTG GAC GAG ATG CTG CGC TCC GAG TAC GGC GGC TCC TTC CGA TTC TCC TCC GAC GCC ATA
Val-Asp-Glu-Met-Leu-Arg-Ser-Glu-Tyr-Gly-Gly-Ser-Phe-Arg-Phe-Ser-Ser-Asp-Ala-Ile-
```

EP 0 170 169 B1

Fig. 6 (C)

```
TCC ACC ACC TTC ACC ACC AAC CTG ACC GAG TAC CCG CTC TCG CGC GTG GAC CTG GGG GAC
Ser - Thr - Thr - Phe - Thr - Thr - Asn - Leu - Thr - Glu - Tyr - Pro - Leu - Ser - Arg - Val - Asp - Leu - Gly - Asp -

TGC ATC GGC AAG GAC GCC CGC GAC GCC ATG GAC CGC ATC TTC GCC CGC AGG TAC AAC GCG
Cys - Ile - Gly - Lys - Asp - Ala - Arg - Asp - Ala - Met - Asp - Arg - Ile - Phe - Ala - Arg - Arg - Tyr - Asn - Ala -

ACG CAC ATC AAG GTG GGC CAG CCG CAG TAC TAC CTG GCC AAT GGG GGC TTT CTG ATC GCG
Thr - His - Ile - Lys - Val - Gly - Gln - Pro - Gln - Tyr - Tyr - Leu - Ala - Asn - Gly - Gly - Phe - Leu - Ile - Ala -

TAC CAG CCC CTT CTC AGC AAC ACG CTC GCG GAG CTG TAC GTG CGG GAA CAC CTC CGA GAG
Tyr - Gln - Pro - Leu - Leu - Ser - Asn - Thr - Leu - Ala - Glu - Leu - Tyr - Val - Arg - Glu - His - Leu - Arg - Glu -

CAG AGC CGC AAG CCC CCA AAC CCC ACG CCC CCG CCG CCC GGG GCC AGC GCC AAC GCG TCC
Gln - Ser - Arg - Lys - Pro - Pro - Asn - Pro - Thr - Pro - Pro - Pro - Gly - Ala - Ser - Ala - Asn - Ala - Ser -

GTG GAG CGC ATC AAG ACA ACC TCC TCC ATC GAG TTC GCC CGG CTG CAG TTT ACG TAC AAC
Val - Glu - Arg - Ile - Lys - Thr - Thr - Ser - Ser - Ile - Glu - Phe - Ala - Arg - Leu - Gln - Phe - Thr - Tyr - Asn -

CAC ATA CAG CGC CAT GTC AAC GAT ATG TTG GGC CGC GTT GCC ATC GCG TGG TGC GAG CTA
His  Ile  Gln  Arg  His  Val  Asn  Asp  Met  Leu  Gly  Arg  Val  Ala  Ile  Ala  Trp  Cys  Glu  Leu

CAG AAT CAC GAG CTG ACC CTG TGG AAC GAG GCC CGC AAG CTG AAC CCC AAC GCC ATC GCC
Gln - Asn - His - Glu - Leu - Thr - Leu - Trp - Asn - Glu - Ala - Arg - Lys - Leu - Asn - Pro - Asn - Ala - Ile - Ala -

TCG GCC ACC GTG GGC CGG CGG GTG AGC GCG CGG ATG CTC GGC GAC GTG ATG GCC GTC TCC
Ser - Ala - Thr - Val - Gly - Arg - Arg - Val - Ser - Ala - Arg - Met - Leu - Gly - Asp - Val - Met - Ala - Val - Ser -

ACG TGC GTG CCG GTT GCC GCG GAC AAC GTG ATC GTC CAA AAC TCG ATG CGC ATC AGC TCG
Thr - Cys - Val - Pro - Val - Ala - Ala - Asp - Asn - Val - Ile - Val - Gln - Asn - Ser - Met - Arg - Ile - Ser - Ser -

CGG CCC GGG GCC TGC TAC AGC CGC CCC CTG GTC AGC TTT CGG TAC GAA GAC CAG GGC CCG
Arg - Pro - Gly - Ala - Cys - Tyr - Ser - Arg - Pro - Leu - Val - Ser - Phe - Arg - Tyr - Glu - Asp - Gln - Gly - Pro -
```

EP 0 170 169 B1

Fig. 6 (D)

```
TTG GTC GAG GGG CAG CTG GGG GAG AAC AAC GAG CTG CGG CTG ACG CGC GAT GCG ATC GAG
Leu-Val-Glu-Gly- Gln-Leu-Gly- Glu-Asn-Asn-Glu-Leu-Arg-Leu-Thr- Arg-Asp-Ala-Ile-Glu-

CCG TGC ACC GTG GGA CAC CGG CGC TAC TTC ACC TTC GGT GGG GGC TAC GTG TAC TTC GAG
Pro-Cys-Thr-Val-Gly-His-Arg-Arg-Tyr-Phe-Thr-Phe-Gly-Gly-Gly-Tyr-Val-Tyr-Phe-Glu-

GAG TAC GCG TAC TCC CAC CAG CTG AGC CGC GCC GAC ATC ACC ACC GTC AGC ACC TTC ATC
Glu-Tyr- Ala-Tyr-Ser-His-Gln-Leu-Ser-Arg-Ala-Asp-Ile-Thr-Thr-Val-Ser-Thr-Phe-Ile-

GAC CTC AAC ATC ACC ATG CTG GAG GAT CAC GAG TTT GTC CCC CTG GAG GTG TAC ACC CGC
Asp-Leu-Asn-Ile-Thr-Met-Leu-Glu-Asp-His-Glu-Phe-Val-Pro-Leu-Glu-Val-Tyr-Thr-Arg-

CAC GAG ATC AAG GAC AGC GGC CTG CTG GAC TAC ACG GAG GTC CAG CGC CGC AAC CAG CTG
His-Glu-Ile-Lys-Asp-Ser-Gly-Leu-Leu-Asp-Tyr-Thr-Glu-Val-Gln-Arg-Arg-Asn-Gln-Leu-

CAC GAC CTG CGC TTC GCC GAC ATC GAC ACG GTC ATC CAC GCC GAC GCC AAC GCC GCC ATG
His-Asp-Leu-Arg-Phe-Ala-Asp-Ile-Asp-Thr-Val-Ile-His-Ala-Asp-Ala-Asn-Ala-Ala-Met-

TTC GCG GGC CTG GGC GCG TTC TTC GAG GGG ATG GGC GAC CTG GGG CGC GCG GTC GGC AAG
Phe-Ala-Gly-Leu-Gly-Ala-Phe-Phe-Glu-Gly-Met-Gly-Asp-Leu-Gly-Arg-Ala-Val-Gly-Lys -

GTG GTG ATG GGC ATC GTG GGC GGC GTG GTA TCG GCC GTG TCG GGC GTG TCC TCC TTC ATG
Val-Val-Met-Gly-Ile-Val-Gly-Gly-Val-Val-Ser-Ala-Val-Ser-Gly-Val-Ser-Ser-Phe-Met-

TCC AAC CCC TTT GGG GCG CTG GCC GTG GGT CTG TTG GTC CTG GCC GGC CTG GCG GCG GCC
Ser-Asn-Pro-Phe-Gly-Ala-Leu-Ala-Val-Gly-Leu-Leu-Val-Leu-Ala-Gly-Leu-Ala-Ala-Ala -

TTC TTC GCC TTT CGT TAC GTC ATG CGG CTG CAG AGC AAC CCC ATG AAG GCC CTG TAC CCT
Phe-Phe-Ala-Phe-Arg-Tyr-Val-Met-Arg-Leu-Gln-Ser-Asn-Pro-Met-Lys-Ala-Leu-Tyr-Pro -

CTA ACC ACC AAG GAG CTC AAG AAC CCC ACC AAC CCG GAC GCG TCC GGG GAG GGC GAG GAG
Leu-Thr-Thr-Lys-Glu-Leu-Lys-Asn-Pro-Thr-Asn-Pro-Asp-Ala-Ser-Gly-Glu-Gly-Glu-Glu-
```

EP 0 170 169 B1

Fig. 6 (E)

```
GGC GGC GAC TTT GAC GAG GCC AAG CTA GCC GAG GCC CGG GAG ATG ATA CGG TAC ATG GCC
Gly-Gly-Asp-Phe-Asp-Glu-Ala-Lys-Leu-Ala-Glu-Ala-Arg-Glu-Met-Ile-Arg-Tyr-Met-Ala-

CTG GTG TCT GCC ATG GAG CGC ACG GAA CAC AAG GCC AAG AAG AAG GGC ACG AGC CGG CTG
Leu-Val-Ser-Ala-Met-Glu-Arg-Thr-Glu-His-Lys-Ala-Lys-Lys-Lys-Gly-Thr-Ser-Arg-Leu-

CTC AGC GCC AAG GTC ACC GAC ATG GTC ATG CGC AAG CGC CGC AAC ACC AAC TAC ACC CAA
Leu-Ser-Ala-Lys-Val-Thr-Asp-Met-Val-Met-Arg-Lys-Arg-Arg-Asn-Thr-Asn-Tyr-Thr-Gln-

GTT CCC AAC AAA GAC GGT GAC GCC GAC GAG GAC GAC CTG TGA CGGGGGGTTTGTTGTA
Val-Pro-Asn-Lys-Asp-Gly-Asp-Ala-Asp-Glu-Asp-Asp-Leu-

TAAATAAAAACCACGGGTGTTAAACCGCATGCGCATCTTTTGGTTTTTTTGTTTGGTCAGCCTTTTGTGTGT

GTGTGGGAAGAAAGAAAAAGGAACACATAAAACTCCCCCGGG
```

EP 0 170 169 B1

Fig. 8

2μori
ars1
leu2
Apr
pAM82(Sac)
Acid phosphatase promoter
Sac I

Fig. 9

ars1
2μori
Apr
leu2
pONYGB
BamHI
gB gene
Acid phosphatase promoter
XhoI

Fig. 10

Fig. 11

Fig. 12

Acid
phosphatase
promoter

SacI

pAMGB1

SacI

gB gene

| ACC | ACC | AAG | GAG | CTC | GTC | GAG | GGG | GCA | TGA | CTA | TCG |
|-----|-----|-----|-----|-----|-----|-----|-----|-----|-----|-----|-----|
| Thr | Thr | Lys | Glu | Leu | Val | Glu | Gly | Ala | stop | | |
| 812 | 813 | 814 | 815 | 816 | 817 | 818 | 819 | 820 | | | |

————— gB gene ——————→  ——————————— Vector gene